# EUROPÄISCHE PATENTANMELDUNG

(11) **EP 0 875 257 A1**
(43) Veröffentlichungstag der Anmeldung: **04.11.1998**
(21) Anmeldenummer: 97107060.2
(22) Anmeldetag: 29.04.1997
(51) Int. Cl.: A61M 1/06

(54) **Kolbenpumpe für Brustabsaugeinrichtung**

(71) Anmelder: Medela AG, 6340 Baar (CH)
(72) Erfinder: Moser, Beat, 6330 Cham (CH); Huber, Tony, 6300 Zug (CH)
(74) Vertreter: Troesch Scheidegger Werner AG

(57) **Zusammenfassung**

Die Kolbenpumpe ist für verschieden grosse Arbeitshübe einstellbar, indem am Kolbenschaft (13) mehrere Nuten (16, 17) unterschiedlicher Länge vorgesehen sind, in welche ein am Zylinder (10) angeordneter, radial nach innen ragender Anschlagnocken (18) durch relative Verdrehung von Zylinder (10) und Kolbenschaft (13) wahlweise einrastbar ist.

## Beschreibung

Die vorliegende Erfindung betrifft eine Kolbenpumpe für eine Brustabsaugeinrichtung, mit einem Zylinder, aus dessen Boden ein Anschluss für eine Absaughaube mündet und an dessen hinterem Ende ein Entlüftungsloch vorgesehen ist, ferner mit einem Kolbenschaft mit am vorderen Ende aufgesetzter, gummielastischer Dichtung, welcher im Zylinder zwischen zwei den Hub und damit den zu erzeugenden Vakuumlevel begrenzenden Endanschlägen hin- und herbewegbar ist.

Derartige, in der Regel von Hand zu betätigende Kolbenpumpen sind bekannt und haben sich neben aufwendigen elektrisch betriebenen Absaugpumpen gut bewährt.

Das Einstellen des gewünschten Vakuumlevels erfolgt dabei rein intuitiv durch mehr oder weniger grosse Hubbewegungen. Dieses Vorgehen ist stark von der Geschicklichkeit der die Pumpe betätigenden Person abhängig und somit zumindest nicht sehr komfortabel.

Aufgabe der vorliegenden Erfindung war es nun, bei einer Kolbenpumpe der eingangs beschriebenen Art mit dem Aufbau der Pumpe mit entsprechenden, einfachen Mitteln eine Einstellbarkeit des Vakuumlevels bzw. der Saugleistung pro Hub zu schaffen, indem eine wählbare bzw. einstellbare Hubbegrenzung vorzusehen ist.

Diese Aufgabe wurde bei einer Kolbenpumpe der eingangs definierten Art erfindungsgemäss durch die Merkmale gemäss dem kennzeichnenden Teil von Anspruch 1 gelöst.

Diese Konstruktion ermöglicht durch einfaches Verdrehen des Kolbenschaftes bezüglich des Zylinders, unter Aufwendung einer nur geringen Kraft, die gewünschte Saugleistung einzustellen.

Besondere Ausführungsformen des Erfindungsgegenstandes sind in den abhängigen Ansprüchen definiert.

Die Erfindung wird nachstehend anhand von in der Zeichnung dargestellten Ausführungsbeispielen noch etwas näher erläutert. Es zeigen:
- Fig. 1: eine Gesamtansicht einer Brustabsaugeinrichtung mit getrennt von der Absaughaube angeordneter Kolbenpumpe;
- Fig. 2: eine Brustabsaugeinrichtung mit direkt an der Absaughaube angeordneter Pumpe;
- Fig. 3 und 4: rein schematisch eine erfindungsgemässe Pumpe in der Stellung nach dem Saughub bzw. direkt vor dem Saughub;
- Fig. 5: einen erfindungsgemäss ausgebildeten Kolbenschaft im Längsschnitt;
- Fig. 6: einen Querschnitt durch die Linie VI-VI von Fig. 5;
- Fig. 7: einen Längsschnitt durch einen Pumpenzylinder;
- Fig. 8: einen Querschnitt durch die Linie VIII-VIII von Fig. 7;
- Fig. 9: ein auf den Zylinder nach Fig. 7 aufsetzbares Ringsegment mit Anschlagnocken;
- Fig. 10: eine Ansicht von unten auf das Ringsegment von Fig. 9;
- Fig. 11: entsprechend der Fig. 4, eine Variante einer erfindungsgemässen Pumpe und
- Fig. 12: einen Querschnitt durch den Kolbenschaft von Fig. 11, entlang der Linie XII-XII.

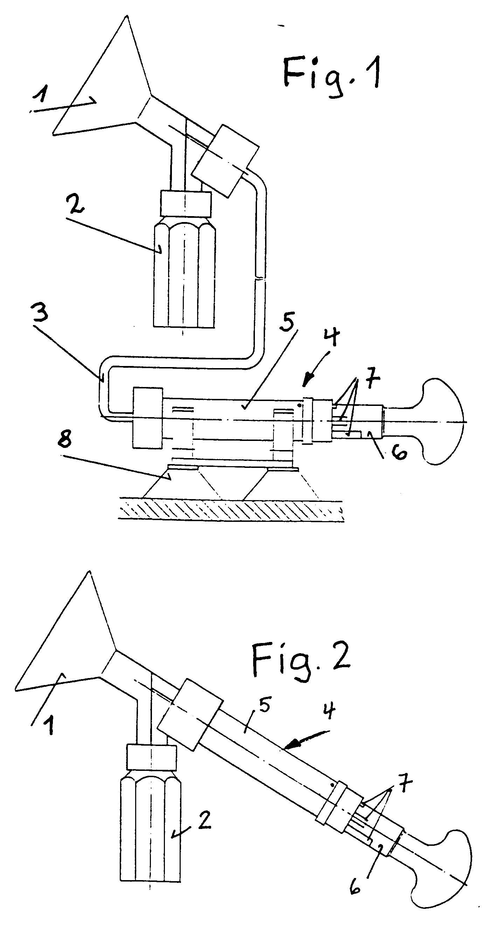

Fig. 1 der Zeichnung zeigt eine Brustabsaugeinrichtung, mit Absaughaube 1 und daran angeschraubter Flasche 2, welche über einen Verbindungsschlauch 3 an eine erfindungsgemässe Kolbenpumpe 4 mit einstellbarem Vakuumlevel angeschlossen ist. Die Pumpe 4 besteht aus einem Zylinder 5 und einem darin hin- und herbewegbaren Kolbenschaft 6. Die für die Verstellbarkeit wichtigen Nuten 7 auf dem Kolbenschaft 6 werden weiter unten noch näher erläutert. Die Pumpe 4 ist in einer Tischhalterung 8 abnehmbar (einschnappbar) befestigt.

Fig. 2 zeigt eine ähnliche Einrichtung wie Fig. 1, jedoch mit direkt an der Absaugeinrichtung 1 montierter Pumpe 4.

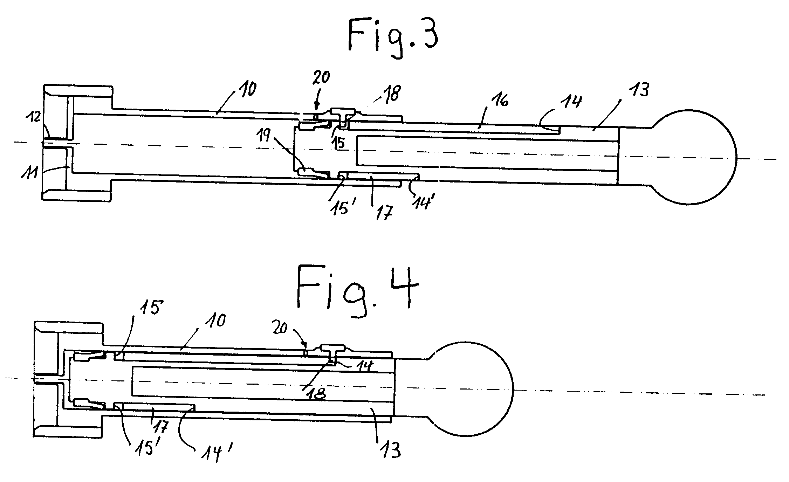

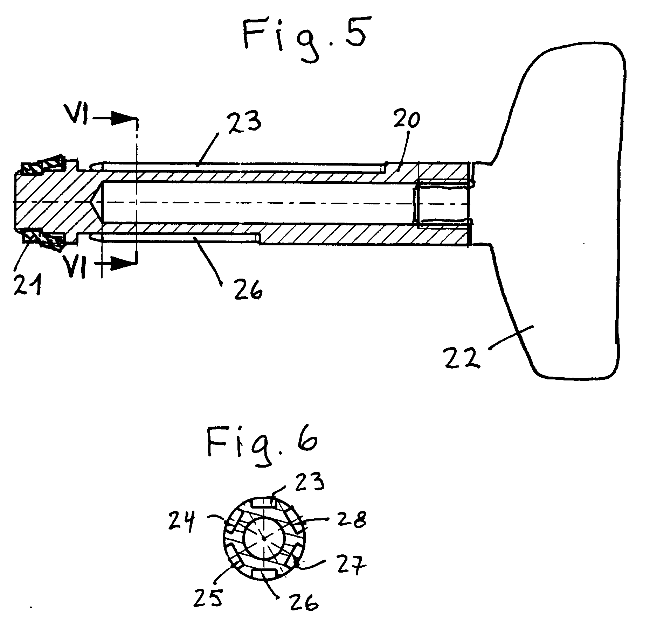

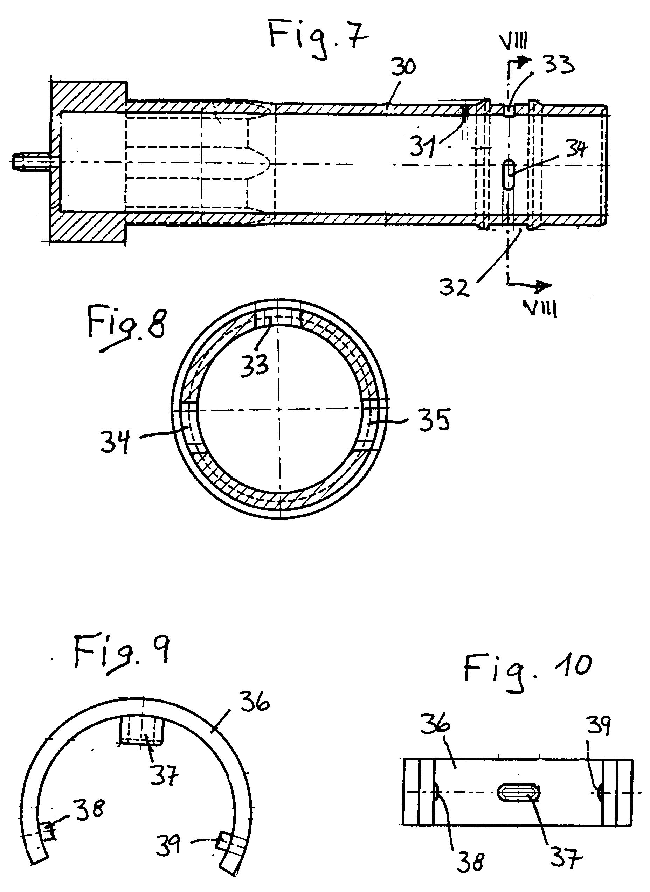

Fig. 3 und 4 zeigen rein schematisch eine erfindungsgemäss aufgebaute Kolbenpumpe: Im Zylinder 10, aus dessen Boden 11 ein Verbindungsanschluss 12 zu einer Absaughaube (nicht gezeigt) mündet, kann ein Kolbenschaft 13 zwischen zwei Endanschlägen 14, 15 bzw. 14', 15' hin- und herbewegt werden. Letztere sind an den Enden von Längsnuten 16, 17 auf der Schaftoberfläche gebildet, wobei ein am Zylinder 10 angebrachter, radial nach innen ragender Anschlagnocken 18 in der Nute 16 geführt ist und die Bewegung (Hub) begrenzt.

Der Kolbenschaft 13 trägt eine Dichtung 19 aus gummielastischem Material (z.B. Silikon).

Am hinteren Ende bzw. im hinteren Bereich des Zylinders ist ein Entlüftungsloch 20 vorgesehen: Wenn aus der Stellung gemäss Fig. 3 der Kolben nach links bewegt wird (bei aufgesetzter Absaughaube), wird die sich komprimierende Luft über die Dichtung 19 entweichen. Beim eigentlichen Saughub, d.h. der Kolbenbewegung aus Fig. 4 nach rechts, entweicht die Luft durch das Entlüftungsloch 20.

Am Umfang des Kolbenschaftes 13 sind mehrere, z.B. sechs, Nuten 16 etc. angeordnet, wobei diese unterschiedlichen Länge aufweisen. Durch Einbringen des Anschlagnockens 18 in eine der Nuten lässt sich somit der Hub und damit der gewünschte Vakuumlevel einstellen. Die Einstellung erfolgt durch Verdrehung des Kolbenschaftes 13 bezüglich des Zylinders 10. Dies wird z.B. dadurch ermöglicht, dass der Anschlagnocken 18 elastisch zurückweicht und immer in die angesteuerte Nute einrastet.

Fig. 5 zeigt einen Kolbenschaft 20 im Schnitt und im Detail. Am Schaft 20 ist neben der Dichtung 21 noch ein Betätigungsgriff 22 montiert.

Wie insbesondere aus Fig. 6 hervorgeht, sind beim gezeigten Beispiel sechs Nuten 23 - 28 unterschiedlicher Länge vorgesehen, welche das Einstellen von sechs verschiedenen Saughublängen erlauben.

Fig. 7 und 8 zeigen den Pumpenzylinder im Detail. Der Zylinder 30 weist am hinteren Ende neben dem Entlüftungsloch 31 insbesondere eine Umfangrille 32 auf, in welcher drei Durchtrittsöffnungen 33, 34, 35 vorgesehen sind (siehe Fig. 8), welche der Aufnahme des Ringsegmentes 36 gemäss Fig. 9 und 10 dienen.

Dieses federnde Ringsegment 36 trägt neben dem eigentlichen Anschlagnocken 37 noch zwei kleine zylindrische Erhöhungen 38, 39. Der Nocken 37 wird durch die Öffnung 33 nach innen in eine der Schaftnuten ragen, während die Erhöhungen 38, 39 in die Öffnungen 34, 35 eingreifen und beim Auffedern des Ringsegments 36 (beim Einstellen der Pumpe) diesen daran hindern wegzuspringen.

Fig. 11 und 12 zeigen eine Variante der erfindungsgemässen Pumpe. Auch hier ist in einem Pumpengehäuse 40 ein Kolbenschaft 41 geführt. Anstelle der Führungsnuten sind bei dieser Variante Kämme 42, 43 unterschiedlicher Länger zur Hubeinstellung vorgesehen. Anstelle des Anschlagnockens ist hier ein den ausgewählten Kamm 42 übergreifender Bügel 44 vorgesehen, welcher am vorderen Ende des Kolbenschaftes 41 durch Verdrehen des letzteren auf den Kamm mit der gewünschten Länge (Hubbegrenzung) gebracht werden kann. Am hinteren Ende jedes Kammes 42, 43 ist dieser unter Bildung eines Anschlages 42' für den Bügel 44 leicht verbreitert.

## Patentansprüche

1. Kolbenpumpe für eine Brustabsaugeinrichtung, mit einem Zylinder, aus dessen Boden ein Anschluss für eine Absaughaube mündet und an dessen hinterem Ende ein Entlüftungsloch vorgesehen ist, ferner mit einem Kolbenschaft mit am vorderen Ende aufgesetzter, gummielastischer Dichtung, welcher im Zylinder zwischen zwei den Hub und damit den zu erzeugenden Vakuumlevel begrenzenden Endanschlägen hin- und herbewegbar ist, dadurch gekennzeichnet, dass zur Hubbegrenzung am Zylinder ein radial nach innen ragendes Folgeorgan vorgesehen ist und am Umfang des Kolbenschaftes verteilt mehrere längsverlaufende Führungen von jeweils unterschiedlicher Länge vorgesehen sind und das Folgeorgan mit einer der Führungen zusammenwirkt, wobei zum Einstellen des gewünschten Hubes der Kolbenschaft relativ zum Zylinder verdrehbar und damit das Folgeorgan mit einer beliebig wählbaren Führung in Wirkverbindung bringbar ist.

2. Kolbenpumpe nach Anspruch 1, dadurch gekennzeichnet, dass die Führungen als Kämme ausgebildet sind und das Folgeorgan den ausgewählten Kamm bügelartig übergreift.

3. Kolbenpumpe nach Anspruch 1, dadurch gekennzeichnet, dass die Führungen als Nuten ausgebildet sind und das Folgeorgan als ein in die ausgewählte Nut eingreifender Anschlagnocken ausgebildet ist.

4. Kolbenpumpe nach Anspruch 3, dadurch gekennzeichnet, dass der Anschlagnocken elastisch federnd in der Zylinderwand angeordnet ist.

5. Kolbenpumpe nach Anspruch 3, dadurch gekennzeichnet, dass der Anschlagnocken auf einem elastisch verbiegbaren Ringsegment angeordnet und auf den Zylinder aufgesetzt durch einen Durchgang nach innen in eine der Nuten ragt.

6. Kolbenpumpe nach einem der Ansprüche 1 bis 5, dadurch gekennzeichnet, dass sie direkt an eine Absaughaube anschliessbar ist.

7. Kolbenpumpe nach einem der Ansprüche 1 bis 5, dadurch gekennzeichnet, dass sie in einer Tischhalterung befestigbar ist und der Anschluss an die Absaughaube über einen Verbindungsschlauch erfolgt.
